(19) Europäisches Patentamt / European Patent Office / Office européen des brevets

(11) **EP 3 990 692 B1**

(12) **EUROPEAN PATENT SPECIFICATION**

(45) Date of publication and mention
of the grant of the patent:
**24.07.2024   Bulletin 2024/30**

(21) Application number: **20830698.5**

(22) Date of filing: **03.06.2020**

(51) International Patent Classification (IPC):
*D21C 11/00* (2006.01)    *D21C 1/02* (2006.01)
*D21C 1/04* (2006.01)    *C08B 1/00* (2006.01)
*C08B 16/00* (2006.01)    *D21C 3/22* (2006.01)

(52) Cooperative Patent Classification (CPC):
**C08B 37/0003; C08B 37/0057; C08H 8/00;
D21C 1/02; D21C 3/22**

(86) International application number:
**PCT/SE2020/050557**

(87) International publication number:
**WO 2020/263153 (30.12.2020 Gazette 2020/53)**

(54) **METHOD FOR EXTRACTING HEMICELLULOSE FROM LIGNOCELLULOSIC MATERIAL**

VERFAHREN ZUR EXTRAKTION VON HEMICELLULOSE AUS LIGNOCELLULOSISCHEM MATERIAL

PROCÉDÉ D'EXTRACTION D'HÉMICELLULOSE D'UNE MATIÈRE LIGNOCELLULOSIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.06.2019   SE 1950785**

(43) Date of publication of application:
**04.05.2022   Bulletin 2022/18**

(73) Proprietor: **VALMET AB
851 94 Sundsvall (SE)**

(72) Inventors:
• **LAMMI, Lari
282 20 Pori (FI)**
• **KARVONEN, Jouni
284 50 Vanha-Ulvila (FI)**

• **MINNAAR, Susanna
0081 Pretoria (ZA)**
• **LANDMAN, Hunphrey
1200 Nelspruit (ZA)**
• **COETZEE, Berdine
0081 Pretoria (ZA)**
• **WAUTS, Johann
1201 Nelspruit (ZA)**

(74) Representative: **Novagraaf Group
Chemin de l'Echo 3
1213 Onex / Geneva (CH)**

(56) References cited:
EP-A1- 3 018 251          EP-A1- 3 026 171
WO-A1-2007/090925    WO-A1-2011/110746
WO-A1-2017/142445    CA-A1- 2 368 872
JP-A- 2015 067 918       US-A1- 2015 184 259

EP 3 990 692 B1

**Description**

TECHNICAL FIELD

The present invention relates to a method for extracting hemicellulose from lignocellulosic material wherein lignocellulosic material undergoes hydrolysis in a digester and a first liquid is supplied to and drained from the digester in order to extract hemicellulose.

BACKGROUND

During production of dissolving pulp from lignocellulosic material it is generally desirable to remove hemicellulose in order to obtain a pulp with a high content of alpha-cellulose. This is also done for various other kinds of specialty chemical pulp.

The removal is performed by a hydrolysis step where a treatment liquor such as steam or water is introduced, and hemicellulose is hydrolysed from the wood chips during acid conditions. Afterwards, the slurry thus created is neutralized by an alkaline neutralizing liquor to produce a neutralized hydrolysate. Hemicellulose is broken down by the contact with alkali into smaller components and is removed from the slurry in subsequent washing stages during the pulp production.

In some applications, it is now desirable to preserve the hemicellulose in hydrolysate and remove it from the slurry for further use in other fields of industry such as the food industry, livestock industry, or biofuels. However, only a small part of available hemicellulose is removed using prior art methods. It is also difficult to keep the hemicellulose separate from the alkali added to the hydrolysate and used for neutralization, and to remove and preserve hemicellulose without lowering the efficiency of the pulp process.

Since the economic value of the finished pulp is at the moment significantly higher than that of the removed hemicellulose, any commercially viable process for extracting and removing hemicellulose cannot be allowed to impair the pulping process.

There is therefore a need for a method for extracting hemicellulose from lignocellulosic material that takes the above considerations and drawbacks into account and that enables removal of hemicellulose in a more efficient way while at the same time having a minimal impact on the process for producing pulp.

SUMMARY

The object of the present invention is to eliminate or at least to minimize the problems discussed above. This is achieved by a method for extracting hemicellulose from lignocellulosic material according to the appended independent claims.

Thus, the method for extracting hemicellulose from lignocellulosic material according to the present invention comprises:

a) providing (11) lignocellulosic material in a digester,

b) supplying (12) steam to the digester,

c) performing (13) hydrolysis of the lignocellulosic material in the digester until a first P-factor is reached, wherein the first P-factor is 50-1000,

d) supplying (14) a first liquid to the digester for mixing with the lignocellulosic material, wherein a total volume of the first liquid is at least equal to a volume required to cover the lignocellulosic material in the digester after the hydrolysis of step c), wherein the first liquid is water or condensate, wherein the first liquid has a temperature of at least 100 °C when it is supplied to the digester, and wherein the supplying of liquid takes place at a flow rate of at least 250 l/s,

e) draining (15) the first liquid from the digester for extracting hemicellulose from the lignocellulosic material, wherein the draining of the first liquid takes place at a flow rate of at least 150 l/s,

wherein the supplying of liquid in step d) takes less than 15 minutes, preferably less than 10 minutes, and wherein

the draining of the first liquid in step e) is performed in less than 15 minutes, preferably less than 10 minutes.

Thereby hemicellulose that has been released from the lignocellulosic material during hydrolysis is removed by the supply and draining of the first liquid so that hemicellulose is extracted from the digester in an efficient and time saving manner. Since hemicellulose is generally broken down into smaller molecules over time the efficient removal from the digester also aids in preserving the hemicellulose. The pulping process can then also proceed with neutralizing of contents in the digester without undue delay which is beneficial from a cost perspective.

A total volume of the first liquid in step d) is at least equal to a volume required to cover the lignocellulosic material in the digester after the hydrolysis of step c). By the first liquid covering the lignocellulosic material completely the extraction of hemicellulose is further improved. It is advantageous to limit the volume of the first liquid to a volume that covers the lignocellulosic material but that does not completely fill the digester so that the fluid consumption can be kept low.

The draining of the first liquid in step e) may be performed through at least one screen in a lower part of the digester. Thereby, the first liquid is drained efficiently and the screen prevents pulp from being discharged along with the first liquid and the hemicellulose present in the first liquid. The screen may suitably be a bar screen or hole type of screen in order to allow for a high flow rate during draining.

A free volume in the digester after the lignocellulosic material is provided in step a) may suitably be 50-70 % of a total volume in the digester, preferably 60-65 % of the total volume. This provides a suitable amount of lignocellulosic material so that hydrolysis can be performed to the desired p-factor.

The supplying of liquid in step d) takes place at a flow rate of at least 250 l/s, preferably at least 300 l/s and more preferably at least 400 l/s. Thereby, the filling of the digester to a level where the lignocellulosic material is covered with the first liquid takes only 15 minutes or less.

The first liquid has a temperature of at least 100 °C, preferably at least 130 °C and more preferably at least 160 °C when it is supplied to the digester in step d). Thereby a lowering of temperature after the hydrolysis can be avoided, and by maintaining the lignocellulosic material at an elevated temperature time can also be saved since a re-heating of the lignocellulosic material after removal of hemicellulose is not needed.

The first liquid is water or condensate, i.e. steam that has been allowed to condense. Thereby the first liquid will have a neutral pH (or the pH is lowered with e.g. SO2 addition or some acid) and will raise the pH of the lignocellulosic material while at the same time extracting hemicellulose. The water can also be recovered after hemicellulose has been removed and reused for the same purpose or for other purposes during the pulping process.

A second liquid may be supplied during the draining of the first liquid in step e), said second liquid preferably being water or a neutralizing liquor. By supplying a second liquid at the same time as the first liquid is drained the process is rendered even more time efficient. The second liquid can improve draining of the first liquid by displacing the first liquid. If the second liquid is a neutralizing liquor that has a higher pH, such as white liquor for instance, neutralizing of the lignocellulosic material can be initiated already during draining of the first liquid to further save time.

The first P-factor is 50-1000, preferably 100-800. In some embodiments, the first P-factor may be selected so that hydrolysis is completed before the first liquid is supplied, but in other embodiments this may take place earlier so that hydrolysis progresses also during supply of the first liquid in order to further save time and so that hydrolysis is finished by the time that the first liquid is drained from the digester.

The first liquid is drained at a flow rate of at least 150 l/s during step e), more preferably at least 200 l/s. Thereby, the draining is also performed in a time efficient way to further save time for the pulping process. Thus, the draining of the first liquid during step e) is performed in less than 15 minutes, preferably less than 10 minutes.

Many additional benefits and advantages of the present invention will be readily understood by the skilled person in view of the detailed description below.

DRAWINGS

The invention will now be described in more detail with reference to the appended drawings, wherein

Fig. 1 discloses schematically a method according to a preferred embodiment of the present invention; and

Fig. 2 discloses a planar view from the side of a digester suitable for extracting hemicellulose according to the invention.

DETAILED DESCRIPTION

The term lignocellulosic material is used herein to mean materials containing lignin, cellulose and hemicellulose. One example of such materials is wood, others include other agricultural or forestry wastes. The lignocellulosic material is commonly divided into small pieces, chips or fragments before the pulping process is initiated.

As used herein, the term P-factor signifies how far a hydrolysis has progressed and is determined using the following formula, where T is a temperature in Kelvin and t is a treatment time in hours.

$$P - factor = \int_0^t \frac{k(T)}{k_{100°C}} dt = \int_0^t e^{40.48 - \frac{15106}{T}} dt$$

The present invention relates to the production of dissolving pulp from a lignocellulosic material, but it is to be noted that what is said below with reference to dissolving pulp is also applicable to other types of specialty pulp or pulp production in general where it is desirable to remove hemicellulose.

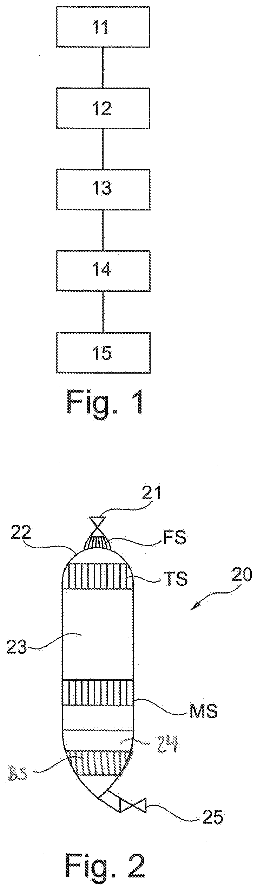

The invention will now be described in detail with reference to Fig. 1 and Fig. 2, showing a method for extracting hemicellulose and a digester for batch cooking of lignocellulosic material, respectively.

Pulping processes are well known within the art and will not be described in detail herein where it does not particularly relate to the present invention. Suffice it to say that a lignocellulosic material is placed in a digester together with a treatment liquid and a hydrolysis is performed at acidic conditions to form a pulp. Following a neutralization, the pulp is cooked at alkaline conditions to arrive at a finished pulp.

Fig. 2 discloses an assembly according to a preferred embodiment of the present invention, comprising a digester 20 having a top 22, a middle 23 and a bottom 24. Also provided in the digester 20 are an upper inlet 21 and a lower inlet 25, along with a plurality of screens for inserting and/or withdrawing liquid to and from the treatment vessel 2. In this embodiment, there are a feed screen FS, a top screen TS, a middle screen MS and a bottom screen BS but alternatively there could be fewer or more screens located at the same or other parts of the digester 20. The feed screen FS may alternatively be used as a degassing screen to control a pressure in the digester and to release non condensable gases. Pumps may be located in withdrawal lines from any or all of the screens but are not shown in the Figure. The digester 20 is in the preferred embodiment a batch digester so that both hydrolysis and cooking take place in the same treatment vessel but in other embodiments other digesters could instead be used.

According to the preferred embodiment of the present invention, lignocellulosic material is provided 11 to the digester 20, preferably by feeding into the digester 20, preferably through the upper inlet 21. Then, steam is supplied 12 to the digester 20, preferably through the feed screen FS but alternatively through the bottom screen BS or middle screen MS or any other suitable inlet, and a hydrolysis is performed 13 of the lignocellulosic material until a desired P-factor is reached. In this embodiment, the hydrolysis is at a temperature of 165-170 °C but other temperatures could alternatively be used. The steam temperature is generally higher than the hydrolysis temperature. It is advantageous from an economical perspective to add the steam at first a lower pressure of about 2.5-4 bar and change when the temperature rises to a higher pressure steam of about 10-12 bar. Other pressure intervals may also be suitable.

Generally, the top screen TS is used for withdrawing displaced liquors and also for withdrawing gases. The middle screen MS is used for withdrawing and circulating cooking liquor during cooking of the lignocellulosic material.

Typically, steam flow to the digester is as fast as possible to reduce the heating time or to get to a target hydrolysis temperature of 160-170 °C. This takes 30-60 min typically. The P-factor range depends on the raw material and the product to be produced but is 200-800 commonly.

In some embodiments, a target P-factor is obtained before hemicellulose is removed but in other embodiments the target P-factor may instead be reached during removal of hemicellulose.

When the desired P-factor is reached a first liquid is supplied 14 to the digester for mixing with the lignocellulosic material, preferably through the upper inlet 21 or the lower inlet 25. Suitably, a total volume of first liquid is a volume that is required to at least cover the lignocellulosic material. After hydrolysis, the lignocellulosic material generally takes up a smaller amount of the total volume in the digester 20 compared to before, usually about 60-80% of the volume required for the lignocellulosic material before hydrolysis is initiated. It is advantageous to minimize the total volume of first liquid to also minimize the need for recovery of the first liquid after hemicellulose has been extracted. Thus, the total volume of first liquid is preferably a volume that is about 40-50 % of a total digester volume in the digester after hydrolysis. A free volume in the digester after the lignocellulosic material is provided in step a) may be about 65 % of the total volume in the digester, preferably 60-65 % of the total volume.

To significantly decrease the time required for supplying the first liquid to the digester, at least two inlets can be used simultaneously.

A batch digester may have a volume of about 100-500 m$^3$ in order to treat a volume of 20-150 ton of dry lignocellulosic material, but the capacity may of course vary depending on technical as well as commercial factors for each batch digester.

In the preferred embodiment, the first liquid is water but other fluids could alternatively be used such as acid reinforced water, SO2 pH adjusted water, or condensates from evaporation or sugar refining steps of the pulping process. The flow rate when supplying 14 the first liquid to the digester 20 is at least 250 l/s, preferably at least 300 l/s and more preferably at least 400 l/s. This has the advantage that significant time is saved while at the same time sufficient hemicellulose will be extracted from the lignocellulosic material and be drained from the digester 20 together with the first liquid. It is thus also advantageous to supply 14 the first liquid to the digester 20 through the upper inlet 21 or the lower inlet 25 in order to facilitate the high flow rate.

In one embodiment, using a digester that has a total volume of 400 m$^3$, the volume of first liquid could be 160-200 m$^3$. At a flow rate of 250 l/s this would give a total time of 10-13 minutes for supplying the first liquid. For a flow rate of 300 l/s the time for supplying the first liquid would instead be 9-11 minutes. In other words, since the volume and dimensions of a digester are known, and the quantity and volume and behavior of the material in the digester are known, it can readily be determined what flow rate is required to supply a specific liquid volume in a given time; and it can in particular be determined what liquid volume is required to cover the lignocellulosic material in the digester after, e.g., a hydrolysis step. Conversely, the draining rate required to drain the supplied liquid in a specific time can also be readily determined, since the amount of liquid supplied is easily measurable or calculable.

It is very advantageous that the supply of the first liquid takes place at 15 minutes or less, and even more advantageous if it can take place at 10 minutes or less. This is a significant improvement compared to prior art methods where a supply time of 15-30 minutes is commonly required to add a first liquid. In many prior art methods, the first liquid may also be cold or at least significantly colder than the temperature in the digester at or after hydrolysis that is generally 160-170 °C, thereby adding the requirement for a subsequent heating step after removal of hemicellulose so that the lignocellulosic material is once again brought to a suitable process temperature. For the present invention, the first liquid may suitably have a temperature of at least 100 °C, preferably at least 130 °C and more preferably at least 160 °C when it is supplied to the digester.

The first liquid is thus drained 15 from the digester 20 and this will also extract the hemicellulose that has been released from the lignocellulosic material during hydrolysis. The draining 15 of the first liquid preferably takes place through the bottom screen BS or another suitable outlet close to the bottom 24 of the digester 20 such as the outlet 25. It is advantageous to also have a high flow rate when draining the digester 20 in order to make the process even more time efficient. Advantageously, at least two outlets could be used for draining the first liquid. In one embodiment, the middle screen MS could be combined with the bottom screen BS or another outlet at the bottom 24 so that one portion of the first liquid is removed through the middles screen MS at the same time as another portion is removed through the bottom screen BS.

The withdrawal screens are preferably perforated screens or bar type screens and it is advantageous for the screens to be adapted to allow the high flow when draining the digester 20.

There will be some increase of P-factor during the drainage so that a drainage time of 10 min may mean an additional accumulation of P-factor by 67 at 165 °C and by 100 units at 170 °C. In this way, further time may thus be saved by performing the supply and draining of the first liquid before the desired P-factor is reached.

Hemicellulose is drained from the digester 20 together with the first liquid and is transported to subsequent process stages (not shown) where the hemicellulose is separated from the first liquid in a refining or sugar recovery process, as is well known within the art.

The lignocellulosic material in the digester 20 is neutralized by supply of a second liquid that may be water but that is preferably an alkaline liquor such as white liquor that achieves alkaline conditions in the digester 20 so that a subsequent cooking stage can commence. The following process to arrive at a finished dissolving pulp will not be described herein but is already well known to the skilled person.

The draining of the first liquid may advantageously also be performed at a high flow rate so that the draining requires 15 minutes or less, preferably 10 minutes or less. The flow rate for draining may be at least 150 l/s, more preferably at least 200 l/s, and by using more than one outlet for draining or more than one inlet for supplying the first liquid to the digester, a significantly higher flow rate can be achieved.

After draining of the first liquid, a water pad remains in the digester 20 and a second liquid that is a neutralizing liquor such as white liquor or optionally water is added in order to raise the pH in the digester 20 before cooking takes place. After cooking, the pulp is discharged from the digester 20 through the outlet 25. In some embodiments, the second liquid may be supplied during the draining of the first liquid. The second liquid can then be supplied in the upper inlet 21 at the same time as the first liquid is drained through the bottom screen BS or another outlet near the bottom 24 so that a mixing of the second liquid with the first liquid is avoided or takes place only at a small degree. The second liquid may serve to displace the first liquid from the digester 20 so that draining of the first liquid takes place faster and in a more controlled way while preserving the pressure in the digester 20 and avoiding pressure drops which are generally undesirable during pulping processes. Alternatively, the second liquid is supplied after the first liquid has been drained from the digester 20.

**Claims**

1. Method for extracting hemicellulose from lignocellulosic material, the method comprising:

   a) providing (11) lignocellulosic material in a digester,
   b) supplying (12) steam to the digester,
   c) performing (13) hydrolysis of the lignocellulosic material in the digester until a first P-factor is reached, wherein the first P-factor is 50-1000,
   d) supplying (14) a first liquid to the digester for mixing with the lignocellulosic material, wherein a total volume of the first liquid is at least equal to a volume required to cover the lignocellulosic material in the digester after the hydrolysis of step c), wherein the first liquid is water or condensate, wherein the first liquid has a temperature of at least 100 °C when it is supplied to the digester, and wherein the supplying of liquid takes place at a flow rate of at least 250 l/s,
   e) draining (15) the first liquid from the digester for extracting hemicellulose from the lignocellulosic material, wherein the draining of the first liquid takes place at a flow rate of at least 150 l/s
   wherein the supplying of liquid in step d) takes less than 15 minutes, preferably less than 10 minutes, and wherein the draining of the first liquid during step e) is performed in less than 15 minutes, preferably less than 10 minutes.

2. Method according to claim 1, wherein a free volume in the digester after the lignocellulosic material is provided in step a) is 50-70 % of a total volume in the digester, preferably 60-65 % of the total volume.

3. Method according to claim 1 or 2, wherein the draining of the first liquid in step e) is performed through at least one screen in a lower part of the digester.

4. Method according to any previous claim, wherein the supplying of liquid in step d) takes place at a flow rate of at least 300 l/s, preferably at least 400 l/s.

5. Method according to any previous claim, wherein a second liquid is supplied during the draining of the first liquid in step e), said second liquid preferably being water or a neutralizing liquor.

6. Method according to any previous claim, wherein the first P-factor is 100-800.

7. Method according to any previous claim, wherein the draining of the first liquid in step e) takes place at a flow rate of at least 200 l/s.

8. Method according to any previous claim, wherein the first liquid has a temperature of at least 130 °C and preferably at least 160 °C when it is supplied to the digester in step d).

**Patentansprüche**

1. Verfahren zum Extrahieren von Hemicellulose aus lignocellulosischem Material, wobei das Verfahren umfasst:

   a) Bereitstellen (11) von lignocellulosischem Material in einem Kocher,
   b) Zuführen (12) von Dampf zu dem Kocher,
   c) Durchführen (13) einer Hydrolyse des lignocellulosischen Materials in dem Kocher, bis ein erster P-Faktor erreicht ist, wobei der erste P-Faktor 50-1000 beträgt,
   d) Zuführen (14) einer ersten Flüssigkeit zu dem Kocher zum Mischen mit dem lignocellulosischen Material, wobei ein Gesamtvolumen der ersten Flüssigkeit mindestens gleich einem Volumen ist, das erforderlich ist, um das lignocellulosische Material in dem Kocher nach der Hydrolyse von Schritt c) zu bedecken, wobei die erste Flüssigkeit Wasser oder Kondensat ist, wobei die erste Flüssigkeit eine Temperatur von mindestens 100 °C aufweist, wenn sie dem Kocher zugeführt wird, und wobei das Zuführen der Flüssigkeit mit einer Durchflussrate von mindestens 250 l/s erfolgt,
   e) Ablassen (15) der ersten Flüssigkeit aus dem Kocher zum Extrahieren von Hemicellulose aus dem lignocellulosischen Material, wobei das Ablassen der ersten Flüssigkeit mit einer Durchflussrate von mindestens 150 l/s erfolgt
   wobei das Zuführen von Flüssigkeit in Schritt d) weniger als 15 Minuten, vorzugsweise weniger als 10 Minuten dauert, und
wobei das Ablassen der ersten Flüssigkeit während Schritt e) in weniger als 15 Minuten, vorzugsweise weniger als 10 Minuten, durchgeführt wird.

2. Verfahren gemäß Anspruch 1, wobei ein freies Volumen in dem Kocher nach dem Bereitstellen des lignocellulosischen Materials in Schritt a) 50-70 % eines Gesamtvolumens in dem Kocher, vorzugsweise 60-65 % des Gesamtvolumens beträgt.

3. Verfahren nach Anspruch 1 oder 2, wobei das Ablassen der ersten Flüssigkeit in Schritt e) durch mindestens ein Sieb in einem unteren Teil des Kochers erfolgt.

4. Verfahren nach einem der vorstehenden Ansprüche, wobei das Zuführen von Flüssigkeit in Schritt d) mit einer Durchflussrate von mindestens 300 l/s, vorzugsweise mindestens 400 l/s erfolgt.

5. Verfahren nach einem der vorstehenden Ansprüche, wobei während des Ablassens der ersten Flüssigkeit in Schritt e) eine zweite Flüssigkeit zugeführt wird, wobei es sich bei der zweiten Flüssigkeit vorzugsweise um Wasser oder eine Neutralisationsflüssigkeit handelt.

6. Verfahren nach einem der vorstehenden Ansprüche, wobei der erste P-Faktor 100-800 beträgt.

7. Verfahren nach einem der vorstehenden Ansprüche, wobei das Ablassen der ersten Flüssigkeit in Schritt e) mit einer Durchflussrate von mindestens 200 l/s erfolgt.

8. Verfahren nach einem der vorstehenden Ansprüche, wobei die erste Flüssigkeit eine Temperatur von mindestens 130 °C und vorzugsweise mindestens 160 °C aufweist, wenn sie in Schritt d) dem Kocher zugeführt wird.

**Revendications**

1. Procédé d'extraction d'hémicellulose à partir d'une matière lignocellulosique, le procédé comprenant :

a) la fourniture (11) d'une matière lignocellulosique dans un digesteur,
b) l'introduction (12) de vapeur dans le digesteur,
c) la réalisation (13) d'une hydrolyse de la matière lignocellulosique dans le digesteur jusqu'à ce qu'un premier facteur P soit atteint, dans lequel le premier facteur P est compris entre 50 et 1000,
d) l'introduction (14) d'un premier liquide dans le digesteur pour un mélange avec la matière lignocellulosique, dans lequel un volume total du premier liquide est au moins égal à un volume nécessaire pour recouvrir la matière lignocellulosique dans le digesteur après l'hydrolyse de l'étape c), dans lequel le premier liquide est de l'eau ou un condensat, dans lequel le premier liquide a une température d'au moins 100 °C lorsqu'il est introduit dans le digesteur, et dans lequel l'introduction de liquide se fait à un débit d'au moins 250 l/s,
e) l'évacuation (15) du premier liquide du digesteur pour une extraction d'hémicellulose de la matière lignocellulosique, dans lequel l'évacuation du premier liquide se fait à un débit d'au moins 150 l/s
dans lequel l'introduction de liquide à l'étape d) prend moins de 15 minutes, de préférence moins de 10 minutes, et dans lequel l'évacuation du premier liquide pendant l'étape e) est effectuée en moins de 15 minutes, de préférence en moins de 10 minutes.

2. Procédé selon la revendication 1, dans lequel un volume libre dans le digesteur après l'introduction de la matière lignocellulosique à l'étape a) représente 50 à 70 % d'un volume total dans le digesteur, de préférence 60 à 65 % du volume total.

3. Procédé selon la revendication 1 ou 2, dans lequel l'évacuation du premier liquide à l'étape e) est effectuée à travers au moins un tamis dans une partie inférieure du digesteur.

4. Procédé selon l'une quelconque revendication précédente, dans lequel l'introduction de liquide à l'étape d) se fait à un débit d'au moins 300 l/s, de préférence d'au moins 400 l/s.

5. Procédé selon l'une quelconque revendication précédente, dans lequel un second liquide est introduit pendant l'évacuation du premier liquide à l'étape e), ledit second liquide étant de préférence de l'eau ou une liqueur de neutralisation.

6. Procédé selon l'une quelconque revendication précédente, dans lequel le premier facteur P est compris entre 100 et 800.

7. Procédé selon l'une quelconque revendication précédente, dans lequel l'évacuation du premier liquide à l'étape e) se fait à un débit d'au moins 200 l/s.

8. Procédé selon l'une quelconque revendication précédente, dans lequel le premier liquide a une température d'au moins 130 °C et de préférence d'au moins 160 °C lorsqu'il est introduit dans le digesteur à l'étape d).

Fig. 1

Fig. 2